# EUROPÄISCHE PATENTSCHRIFT

(11) **EP 1 557 141 B1**
(45) Veröffentlichungstag und Bekanntmachung des Hinweises auf die Patenterteilung: **30.05.2007**
(21) Anmeldenummer: 04023734.9
(22) Anmeldetag: 09.10.2004
(51) Int. Cl.: A61F 2/18

(54) **Gehörknöchelchenprothese**
Auditory ossicles prosthesis
Prothèse d'osselets de l'oreille

(30) Priorität: 23.01.2004 DE 202004001008 U
(43) Veröffentlichungstag der Anmeldung: 27.07.2005
(73) Patentinhaber: Heinz Kurz GmbH Medizintechnik, 72144 Dusslingen (DE)
(72) Erfinder: Steinhardt, Uwe, 72145 Hirrlingen (DE); àWengen, Dr. Daniel F., 4102 Binningen (CH)
(74) Vertreter: Kohler Schmid Möbus

(56) Entgegenhaltungen:
- DE-U1- 20 014 659
- DE-U1- 20 212 771
- DE-U1- 20 310 609
- FR-A- 2 769 492
- US-A- 3 711 869
- US-A1- 2003 130 734

## Beschreibung

Die Erfindung betrifft eine Gehörknöchelchenprothese die einerseits am Ambossfortsatz der menschlichen Gehörknöchelchenkette und andererseits am Steigbügel befestigt ist oder direkt ins Innenohr getaucht wird.

Gehörknöchelchenprothesen werden verwendet, um bei ganz oder teilweise fehlenden oder geschädigten Gehörknöchelchen des menschlichen Mittelohrs den Schall vom Trommelfell zum Innenohr zu übertragen. Die Gehörknöchelchenprothese weist dabei zwei Enden auf, wobei je nach den konkreten Gegebenheiten das eine Ende der Gehörknöchelchenprothese beispielsweise am Ambossfortsatz der menschlichen Gehörknöchelchenkette befestigt und das andere Ende der Gehörknöchelchenprothese beispielsweise am Steigbügel der menschlichen Gehörknöchelchenkette befestigt oder direkt ins Innenohr getaucht wird.

Da die anatomischen Gegebenheiten des Ohrs, wie beispielsweise die Lage, die Form und die Größe des Steigbügels, des Ambosses, des Hammers und des Trommelfells variieren, ist es vorteilhaft, wenn Gehörknöchelchenprothesen nicht starr ausgebildet sind, sondern eine gewisse Flexibilität oder Variabilität aufweisen. Um diese Flexibilität/Variabilität zu erreichen sind verschiedene Befestigungs- und Ankopplungsvorrichtungen für Gehörknöchelchen, die elastische Teile und/oder Gelenke aufweisen, bekannt. Vielfach wird mit den bekannten Gehörknöchelchenprothesen die Schallleitung zwischen dem Trommelfell und dem Innenohr nur begrenzt ermöglicht, weil sie die natürlichen anatomischen Ausbildungen der Gehörknöchelchenkette nur sehr eingeschränkt ersetzen können.

Aus der DE 203 10 609 U1 ist eine Gehörknöchelchenprothese bekannt, die einerseits einen ersten Clip aufweist, über den sie am Hammergriff der menschlichen Gehörknöchelchenkette befestigbar ist, und die andererseits am Steigbügel befestigbar ist oder direkt ins Innenohr getaucht werden kann, wobei der erste Clip an einem ersten Stab befestigt ist, der in einem Kugelgelenk mit einer Kugel endet, die in einem U-förmigen Pfannenteil gelagert ist, und wobei das Kugelgelenk in einen zweiten Stab übergeht, der als Kolben ausgebildet ist oder in einem zweiten Clip endet. Eine Schall leitende Verbindung zwischen dem Ambossfortsatz der menschlichen Gehörknöchelchenkette und dem Innenohr kann und soll mit dieser bekannten Prothese allerdings nicht hergestellt werden.

Bei den weiter oben diskutierten, bekannten Mittelohrprothesen, die eine solche Überbrückung eines beschädigten oder verkrüppelten Ambossfortsatzes zum Innenohr hin ermöglichen sollen, tritt das Problem auf, dass die bekannten Prothesen abhängig vom Ankoppelpunkt am Ambossfortsatz einen mehr oder weniger erheblichen Teil des Hebelverhältnisses in der Gehörknöchelchenkette verlieren, welches wiederum ein direktes Maß für die Qualität der Schallleitung durch das Mittelohr darstellt.

Aufgabe der vorliegenden Erfindung ist es demgegenüber, eine Gehörknöchelchenprothese der oben beschriebenen Art zu schaffen, mit der die Schallleitung zwischen dem Ambossfortsatz und dem Innenohr verbessert ermöglicht wird, wobei insbesondere die Hebelverhältnisse im Vergleich zum natürlichen Verlauf der Gehörknöchelchenkette im gesunden menschlichen Mittelohr durch den Einsatz der Prothese nicht verschlechtert werden sollen.

Erfindungsgemäß wird diese Aufgabe durch eine Gehörknöchelchenprothese gelöst, die so gestaltet ist, dass sie über den ersten Clip am Ambossfortsatz befestigbar ist und im implantierten Zustand von ihrer Anlenkung am Ambossfortsatz ausgehend den Verlauf des natürlichen Ambossfortsatzes bis zu seinem Ende oder darüber hinaus weitgehend nachbilden und im Bereich der Höhe des natürlichen Endes des Ambossfortsatzes abgewinkelt zum anderen Endpunkt der Gehörknöchelchenprothese am Steigbügel oder am/im Innenohr verlaufen kann, wobei sich die Wirkrichtung (=Öffnungs- und Schließrichtung) des ersten Clip in einer ersten Richtung erstreckt, wobei der erste Clip derart am ersten Stab befestigt ist, dass die Längsachse des ersten Stabes in einer zweiten Richtung transversal zur ersten Richtung verläuft, und wobei im implantierten Zustand der Gehörknöchelchenprothese der zweite Stab über das Kugelgelenk derart gegenüber dem ersten Stab verdreht werden kann, dass die Längsachse des zweiten Stabes parallel zur ersten Richtung verläuft.

Die erfindungsgemäße Gehörknöchelchenprothese hat damit den wesentlichen Vorteil, dass der Übergang vom Ambossfortsatz zum Steigbügel weitestgehend den anatomischen Verhältnissen der natürlichen Gehörknöchelchenkette nachgebildet ist. Die Anbindung der erfindungsgemäßen Prothese am Ambossfortsatz erfolgt ca. 1 mm hinter dem distalen Ende des Fortsatzes und über die Ausrichtung der Prothese in Richtung des Verlaufs des natürlichen Fortsatzes können Hebelverhältnisse erreicht bzw. simuliert werden, die weitestgehend den natürlichen Verhältnissen entsprechen. Die erfindungsgemäße Prothese ist in ihrem Verlauf gelenkig bzw. elastisch abgewinkelt, damit, wie bei der natürlichen Gehörknöchelchenkette, die Schallleitung den Raumverhältnissen im Mittelohr angepasst erfolgen kann. Die Hebelverhältnisse sind bei der erfindungsgemäßen Prothese gegenüber den aus dem Stand der Technik bekannten Prothesen erheblich verbessert, sodass mit der neuen Prothese ein wesentlich verbesserter Hörkomfort erreicht wird.

Die erfindungsgemäße Prothese selbst kann aus gewebe- und knochenverträglichen Kunststoffen, Faserverbundwerkstoffen oder Metallen hergestellt sein, die den abgewinkelten Verlauf der erfindungsgemäßen Prothese in ihrer Beweglichkeit unterstützen bzw. gewährleisten.

Mit dieser gegenständlichen Ausgestaltung der erfindungsgemäßen Gehörknöchelchenprothese wird eine hohe Beweglichkeit erreicht, die die Schallleitung im Mittelohr verbessert unterstützt. Mittels des Kugelgelenks wird eine sehr hohe Beweglichkeit der erfindungsgemäßen Prothese erreicht und dies in einem Verlauf, der der menschlichen Gehörknöchelchenkette nachempfunden ist.

Wird die erfindungsgemäße Prothese über Clips bzw. über einen Kolben am Ambossfortsatz bzw. am Steigbügel befestigt oder über einen Kolben direkt in das Innenohr getaucht, so wird die Flexibilität oder Beweglichkeit der erfindungsgemäßen Prothese nicht behindert.

Eine besonders bevorzugte Ausführungsform eines Gelenks im abgewinkelten Bereich der Prothese wird über eine Kugel und ein U-förmiges Pfannenteil gebildet, in dem die Kugel in den Seitenwänden des Pfannenteils in Öffnungen des Pfannenteils gelagert ist. Über eine derartige konstruktive Ausgestaltung kann sich die Kugel im U-förmig ausgebildeten Pfannenteil uneingeschränkt in alle Richtungen bewegen und Schallleitungen bestmöglich gewährleisten.

In weiterer Ausgestaltung der Erfindung sind der erste und/oder zweite Clip aus zwei V- oder U-förmig angeordneten Federzungen gebildet. Der Halt eines Clips wird über eine derartige Ausgestaltung verbessert. Sind die Kontaktstellen der Clips noch an Stellen aufgeraut, an denen sie am Ambossfortsatz bzw. am Steigbügel aufliegen, so ist eine gesicherte, dauerhafte Befestigung der erfindungsgemäßen Gehörknöchelchenprothese gewährleistet.

Die erfindungsgemäße Gehörknöchelchenprothese kann an einem Ende als Kolben ausgebildet sein, der direkt in das Innenohr eingetaucht wird. Bei dieser Ausführungsform ist ein zweiter Clip nicht notwendig.

Zur erleichterten Platzierung der erfindungsgemäßen Gehörknöchelchenprothese sind an dem ersten und/oder zweiten Clip jeweils ein Haltegriff ausgebildet. Diese Ausgestaltung erleichtert die Anbringung der erfindungsgemäßen Prothese im Mittelohr.

Die erfindungsgemäße Gehörknöchelchenprothese selbst ist aus einem biokompatiblen Material bzw. Materialverbund hergestellt, der Kunststoffteile und/oder auch Metallkomponenten wie Titan, Titanlegierungen oder Nitinol enthalten kann. Die Prothese selbst kann vollkommen aus einem der genannten Metalle gefertigt sein.

Nachfolgend ist die erfindungsgemäße Gehörknöchelchenprothese in einer Ausführungsform in der Zeichnung dargestellt. Die in der Zeichnung dargestellten Ausführungsformen sind beispielhaft zu verstehen und zeigen den erfindungsgemäßen Gegenstand nicht maßstäblich.

Es zeigen:
- **Fig. 1**: eine erfindungsgemäße Gehörknöchelchenprothese in räumlicher Darstellung mit einer Abwinklung, die als Gelenk ausgebildet ist;
- **Fig. 2**: eine Gehörknöchelchenprothese gemäß Fig. 1 aus einem anderen Sichtwinkel;
- **Fig. 3**: einen Ausschnitt einer erfindungsgemäßen Gehörknöchelchenprothese gemäß Fig. 1 und 2, wie sie am Ambossfortsatz angelenkt bzw. befestigt ist.

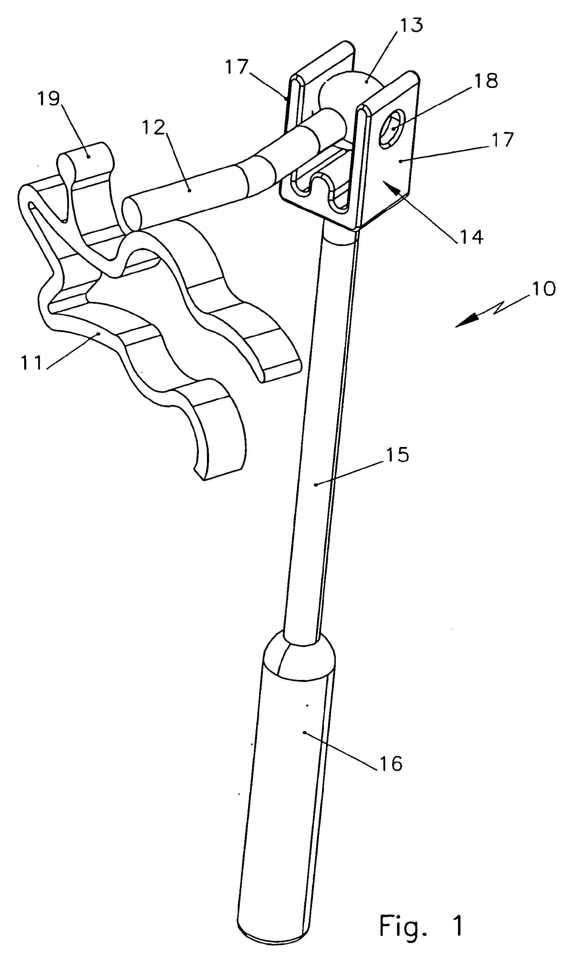

**Fig. 1** zeigt in perspektivischer Darstellung eine erfindungsgemäße Gehörknöchelchenprothese **10**, die einen ersten Clip **11** aufweist, an dem ein erster Stab **12** befestigt ist. Der erste Stab 12 verläuft leicht angewinkelt, er muss nicht geradlinig verlaufen. Der Stab 12 ist materialschlüssig mit dem ersten Clip 11 verbunden, der als V-förmiger Clip mit hoher Flexibilität ausgebildet ist. Der erste Clip 11 wie auch der erste Stab 12 können aus einer Titanlegierung hergestellt sein.

Der erste Stab 12 endet, dem ersten Clip 11 gegenüberliegend, in einer Kugel **13,** die in einem U-förmigen Pfannenteil **14** gelagert ist. An das U-förmige Pfannenteil **14** schließt sich ein zweiter Stab **15** an, der in einen Kolben **16** übergeht. Anstatt des Kolbens 16 kann ein zweiter Clip am zweiten Stab 15 ausgebildet sein. Über den ersten Clip 11 bzw. den Kolben 16 oder den alternativ am Ende des zweiten Stabs 15 ausgebildeten zweiten Clip ist die erfindungsgemäße Gehörknöchelchenprothese 10 im Mittelohr einerseits am Ambossfortsatz und andererseits am Steigbügel bzw. am/im Innenohr gehalten.

Das U-förmige Pfannenteil 14 weist Seitenwände **17** auf, in denen jeweils Öffnungen **18** ausgebildet sind. In diesen Öffnungen 18 ist die Kugel 13 gelenkig gelagert gehalten, sodass eine hohe Beweglichkeit der Gehörknöchelchenprothese 10 zwischen dem ersten Clip 11 und dem Kolben 16 gegeben ist. Am ersten Clip 11 ist ein Haltegriff **19** ausgebildet, über den die Platzierung des ersten Clips 11 am Ambossfortsatz erleichtert wird.

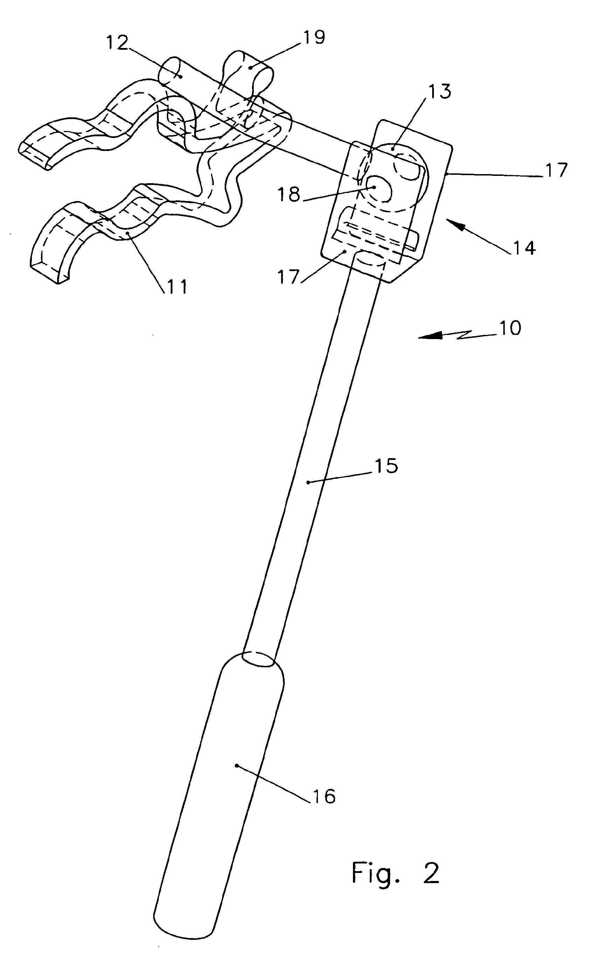

**Fig. 2** zeigt die erfindungsgemäße Gehörknöchelchenprothese 10 aus Fig. 1 in einer weiteren perspektivischen Darstellung aus einem anderen Sichtwinkel, wobei dieselben gegenständlichen Merkmale mit denselben Bezugszeichen versehen sind. Die Anlenkung des ersten Stabs 12 an den zweiten Stab 15 über das U-förmige Pfannenteil 14 ist deutlich zu erkennen und auch die hohe Beweglichkeit der Kugel 13, gelagert in den Öffnungen 18 der Seitenwände 17 des U-förmigen Pfannenteils 14, ist deutlich gezeigt. Ist die erfindungsgemäße Gehörknöchelchenprothese 10 im Mittelohr platziert, so besteht über das Kugelgelenk eine hohe Beweglichkeit zwischen dem ersten Clip 11 und dem Kolben 16.

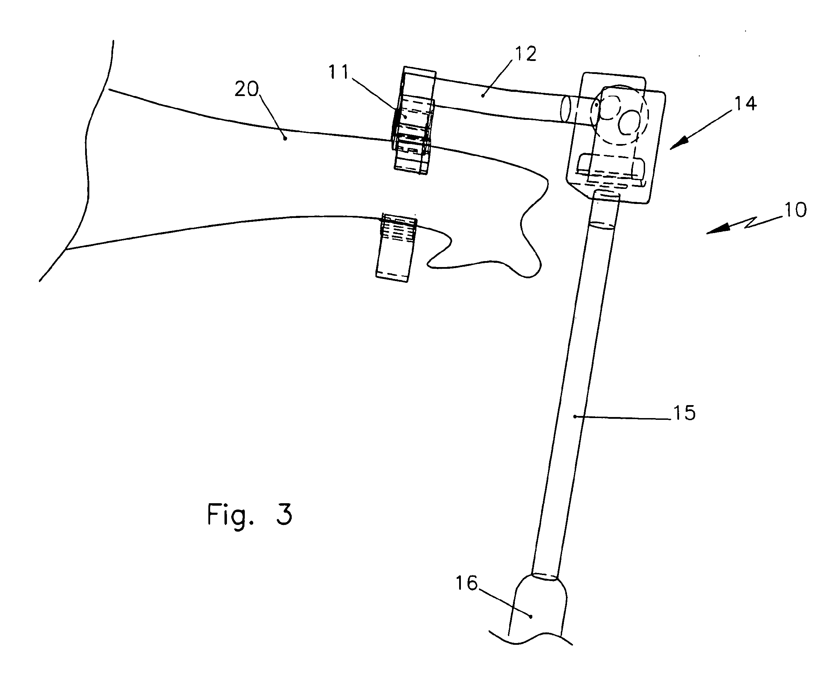

**Fig. 3** zeigt einen Ausschnitt sowohl eines Ambossfortsatzes **20** wie auch der in den Fig. 1 und 2 gezeigten Gehörknöchelchenprothese 10, wie sie am Ambossfortsatz 20 über den ersten Clip 11 befestigt ist. Die Gehörknöchelchenprothese 10 ragt über den natürlichen Ambossfortsatz 20 hinaus, indem der erste Stab 12 gelenkig verbunden in den zweiten Stab 15 übergeht, der in dem noch teilweise gezeigten Kolben 16 endet.

Mit der in den Fig. 1 - 3 gezeigten Gehörknöchelchenprothese 10 wird eine Prothese gezeigt, über die die Hebelverhältnisse bzw. das Tuning des Mittelohrs für Schallleitungen erheblich verbessert werden kann.

## Patentansprüche

1. Gehörknöchelchenprothese (10), die einerseits einen ersten Clip (11) aufweist, über den sie an einem ersten Glied der menschlichen Gehörknöchelchenkette befestigbar ist, und die andererseits am Steigbügel befestigbar ist oder direkt ins Innenohr getaucht werden kann, wobei der erste Clip (11) an einem ersten Stab (12) befestigt ist, der in einem Kugelgelenk mit einer Kugel (13) endet, die in einem U-förmigen Pfannenteil (14) gelagert ist, und wobei das Kugelgelenk in einen zweiten Stab (15) übergeht, der als Kolben (16) ausgebildet ist oder in einem zweiten Clip endet,
**dadurch gekennzeichnet, dass** die Gehörknöchelchenprothese (10) so gestaltet ist, dass sie über den ersten Clip (11) am Ambossfortsatz (20) befestigbar ist und im implantierten Zustand von ihrer Anlenkung am Ambossfortsatz (20) ausgehend den Verlauf des natürlichen Ambossfortsatzes (20) bis zu seinem Ende oder darüber hinaus weitgehend nachbilden und im Bereich der Höhe des natürlichen Endes des Ambossfortsatzes (20) abgewinkelt zum anderen Endpunkt der Gehörknöchelchenprothese (10) am Steigbügel oder am/im Innenohr verlaufen kann, wobei sich die Wirkrichtung, das heißt die Öffnungs- und Schließrichtung des ersten Clip (11) in einer ersten Richtung erstreckt, wobei der erste Clip (11) derart am ersten Stab (12) befestigt ist, dass die Längsachse des ersten Stabes (12) in einer zweiten Richtung transversal zur ersten Richtung verläuft, und wobei im implantierten Zustand der Gehörknöchelchenprothese (10) der zweite Stab (15) über das Kugelgelenk derart gegenüber dem ersten Stab (12) verdreht werden kann, dass die Längsachse des zweiten Stabes (15) parallel zur ersten Richtung verläuft.

2. Gehörknöchelchenprothese nach Anspruch 1, **dadurch gekennzeichnet, dass** der U-förmige Pfannenteil (14) in Seitenwänden (17) Öffnungen (18) aufweist, in denen die Kugel (13) gelagert ist.

3. Gehörknöchelchenprothese nach einem der vorhergehenden Ansprüche, **dadurch gekennzeichnet, dass** der erste Clip (11) und/oder der zweite Clip aus zwei V- oder U-förmig angeordneten Federzungen gebildet ist.

4. Gehörknöchelchenprothese nach einem der vorhergehenden Ansprüche, **dadurch gekennzeichnet, dass** der erste Clip (11) und/oder der zweite Clip an seinen Kontaktstellen zum Ambossfortsatz (20) und/oder zum Steigbügel aufgeraut ist.

5. Gehörknöchelchenprothese nach einem der vorhergehenden Ansprüche, **dadurch gekennzeichnet, dass** der erste Clip (11) und/oder der zweite Clip einen Haltegriff (19) aufweist.

6. Gehörknöchelchenprothese nach einem der vorhergehenden Ansprüche, **dadurch gekennzeichnet, dass** die Prothese oder Teile davon aus Titan, einer Titanlegierung, aus biokompatiblen Kunststoffen oder Faserverbundwerkstoffen oder aus Nitinol hergestellt ist.

## Claims

1. An auditory ossicle prosthesis (10) comprising a first clip (11) enabling it to be fastened to a first limb of the human ossicular chain at one end which prosthesis can be fastened to the stapes or be inserted directly into the inner ear at the other end, whereby the first clip (11) is fastened to a first bar (12) terminating in a ball joint with a ball (13) which is supported in a U-shaped socket part (14), whereby the ball joint transitions into a second bar (15) which terminates as a piston (16) or in a second clip, **characterized in that** the auditory ossicle prosthesis (10) is designed such that it can be fastened to the limb of incus (20) and, being implanted, starting at its articulation on the limb of incus (20), largely can trace the course of the natural limb of incus (20) to its end or past it, and, angled downward in the region of the level of the natural end of the limb of incus (20), can extend to the other endpoint of the auditory ossicle prosthesis (10) on the stapes or at/in the inner ear, whereby the effective direction, i.e. the direction of opening and closing of the first clip (11) extends in a first direction, whereby the first clip (11) is fastened to the first bar (12) such that the longitudinal axis of the first bar (12) runs in a second direction transverse to the first direction, and whereby, in an implanted state of the auditory ossicle prosthesis (10), the second bar (15) is twistable with respect to the first bar (12) by means of the ball joint such that the longitudinal axis of the second bar (15) is running parallel to the first direction.

2. The auditory ossicle prosthesis according to claim 1, **characterized in that** the U-shaped socket part (14) includes openings (18) in side walls (17) in which the ball (13) is supported.

3. The auditory ossicle prosthesis according to anyone of the preceding claims, **characterized in that** the first clip (11) and/or the second clip is formed of two flexible tongues arranged in a V or U-shape.

4. The auditory ossicle prosthesis according to anyone of the preceding claims, **characterized in that** the first clip (11) and/or the second clip is roughened up at its contact points with the limb of incus (20) and/or the stapes.

5. The auditory ossicle prosthesis according to anyone of the preceding claims, **characterized in that** the first clip (11) and/or the second clip includes a handle (19).

6. The auditory ossicle prosthesis according to anyone of the preceding claims, **characterized in that** the prosthesis or parts thereof are made of titanium, a titanium alloy, biocompatible plastics or composite fiber materials, or Nitinol.

## Revendications

1. Prothèse d'osselet de l'oreille (10) qui comporte d'une part un premier clip (11) par lequel elle peut être fixée à un premier élément de la chaîne des osselets de l'oreille humaine, et qui peut être fixée d'autre part à l'étrier ou directement échangée dans l'oreille interne, le premier clip (11) étant fixé à une première barrette (12) qui se termine dans une articulation sphérique avec une sphère (13) qui est montée dans une partie de cavité (14) en U, et l'articulation sphérique se prolongeant par une deuxième barrette (15) qui est réalisée sous la forme d'un piston (16) ou se termine dans un deuxième clip,
**caractérisée en ce que** la prothèse d'osselet de l'oreille (10) est conçue de manière à pouvoir être fixée par le premier clip (11) sur le prolongement d'enclume (20) et, à l'état implanté, elle puisse reproduire, partant de son articulation sur le prolongement d'enclume (20), l'allure du prolongement d'enclume naturel (20) jusqu'à son extrémité ou au-delà, et que dans la zone de la hauteur de l'extrémité naturelle du prolongement d'enclume (20), elle puisse s'étendre, coudée vers l'autre point terminal de la prothèse d'osselet de l'oreille (10), sur l'étrier ou sur/dans l'oreille interne, le sens d'action, c'est-à-dire le sens d'ouverture et de fermeture du premier clip (11) s'étendant dans une première direction, le premier clip (11) étant fixé sur la première barrette (12) de manière que l'axe longitudinal de la première barrette (12) s'étende dans une deuxième direction transversalement à la première direction, et à l'état implanté de la prothèse d'osselet de l'oreille (10), la deuxième barrette (15) pouvant être tournée, à travers l'articulation sphérique, par rapport à la première barrette (12), de manière que l'axe longitudinal de la deuxième barrette (15) s'étende parallèlement à la première direction.

2. Prothèse d'osselet de l'oreille selon la revendication 1, **caractérisée en ce que** la partie de cavité (14) en U présente, dans des parois latérales (17), des ouvertures (18) dans lesquelles est montée la sphère (13).

3. Prothèse d'osselet de l'oreille selon l'une des revendications précédentes, **caractérisée en ce que** le premier clip (11) et/ou le deuxième clip est formé de deux languettes élastiques disposées en V ou en U.

4. Prothèse d'osselet de l'oreille selon l'une des revendications précédentes, **caractérisée en ce que** le premier clip (11) et/ou le deuxième clip est rendu rugueux en ses points de contact avec le prolongement d'enclume (20) et/ou avec l'étrier.

5. Prothèse d'osselet de l'oreille selon l'une des revendications précédentes, **caractérisée en ce que** le premier clip (11) et/ou le deuxième clip comporte une poignée (19).

6. Prothèse d'osselet de l'oreille selon l'une des revendications précédentes, **caractérisée en ce que** la prothèse ou des parties de celle-ci sont fabriquées en titane, en un alliage de titane, en des matières plastiques biocompatibles ou en matériau composite à fibres ou en Nitinol.
